Europäisches Patentamt

European Patent Office

Office européen des brevets

(11) Veröffentlichungsnummer: **0 480 906 B1**

(12) **EUROPÄISCHE PATENTSCHRIFT**

(45) Veröffentlichungstag der Patentschrift: **26.04.95**

(21) Anmeldenummer: **91890236.2**

(22) Anmeldetag: **07.10.91**

(51) Int. Cl.⁶: **A61K 38/48**

(54) **Pharmazeutische Zubereitung auf Basis von Lys-Plasminogen.**

(30) Priorität: **11.10.90 AT 2045/90**

(43) Veröffentlichungstag der Anmeldung:
**15.04.92 Patentblatt 92/16**

(45) Bekanntmachung des Hinweises auf die
Patenterteilung:
**26.04.95 Patentblatt 95/17**

(84) Benannte Vertragsstaaten:
**AT BE CH DE DK ES FR GB IT LI NL SE**

(56) Entgegenhaltungen:
**EP-A- 0 353 218**
**FR-A- 2 474 313**

**Konservative Therapie arterieller Durchblutungsstörungen, G. Trübstein, Bonn 1986,
Seiten 343-351 und 430-432**

(73) Patentinhaber: **IMMUNO Aktiengesellschaft**
**Industriestrasse 67**
**A-1221 Wien (AT)**

(72) Erfinder: **Eibl, Johann, Dr.**
**Gustav Tschermakgasse 2**
**A-1180 (AT)**
Erfinder: **Philapitsch, Anton**
**Hans Kudlichgasse 5**
**A-2490 Ebenfurt (AT)**
Erfinder: **Schwarz, Hans Peter, Dr.**
**Schindlergasse 32**
**A-1180 Wien (AT)**

(74) Vertreter: **Wolfram, Gustav, Dipl.-Ing.**
**Patentanwälte**
**Sonn, Pawloy, Weinzinger & Wolfram,**
**Riemergasse 14**
**A-1010 Wien (AT)**

**Beschreibung**

Die Erfindung betrifft eine pharmazeutische Zubereitung auf Basis von Lys-Plasminogen aus Plasma oder rekombinant hergestelltem Lys-Plasminogen. Diese erfindungsgemäße Zubereitung kann in der Thrombose-Prophylaxe und Thrombose-Therapie verwendet werden.

Ziel jeder medizinischen Behandlung thrombotischer Zustände ist die Wiederherstellung der Gefäßintegrität durch die Auflösung des Thrombus. Das kann erreicht werden, indem im Blut und insbesondere am Thrombus selbst Plasmin - und damit fibrinolytische Aktivität - freigesetzt wird.

Die herkömmliche medizinische Behandlung besteht darin, Substanzen in hoher Dosis in die Blutbahn zu infundieren, die im Blut vorhandenes Plasminogen in Plasmin umwandeln. (Lancet, 1985 I, Seiten 842 bis 847). Als derartige Aktivatoren werden Streptokinase, Urokinase, ein Plasminogen-Streptokinase-Komplex und Tissue-Typ-Plasminogen Aktivator verwendet.

Ein Nachteil aller dieser Aktivatoren ist, daß sie zu schweren Komplikationen beim Patienten führen können, weil sie systemisch wirken und nicht nur jenes Plasminogen aktivieren, das am Thrombus selbst adsorbiert ist (vgl. z. B. Circulation, 1985, Vol. 72, No. 6, Seiten 1321 bis 1326). Dies führt zum Abbau von Fibrinogen und -Gerinnungsfaktoren und schließlich zur hämorrhagischen Diathese.

"Lys-Plasminogen" ist ein in der Literatur verwendeter Sammelbegriff für proteolytisch modifizierte Formen des nativen Plasminogens ( = Glu-Plasminogen), die aus diesem durch Abspaltung eines Polypeptides vom $NH_2$-Terminus erhalten werden. Als N-terminale Aminosäure der heute bekannten Species des Lys-Plasminogens wurden bisher Lysin, Methionin und Valin nachgewiesen. Als Molekulargewicht werden in der Literatur für Glu-Plasminogen Werte zwischen 90.000 und 94.000 und für Lys-Plasminogen Werte von etwa 80.000 angegeben.

Lys-Plasminogen besitzt eine höhere Bindungsaffinität an Thromben als Glu-Plasminogen. Außerdem läßt es sich um ein Vielfaches schneller zu Plasmin aktivieren.

In der Literatur wird auch eine Behandlung mit Lys-Plasminogen beschrieben, das entweder allein oder in Kombination mit einem Aktivator infundiert wird (z.B. in "Konservative Therapie arterieller Durchblutungsstörungen", G. Trübestein, Bonn, 1986, Seiten 343 bis 351 und Seiten 430 bis 432).

Alle heute bekannten Methoden und Präparationen zur Behandlung oder Prophylaxe von Thrombosen und Plasminogenmangelzuständen lassen hinsichtlich ihrer Effizienz noch zu wünschen übrig. Der thrombolytische Effekt könnte durch Verabreichung von Lys-Plasminogen gesteigert werden.

Die Erfindung stellt sich daher die Aufgabe, eine pharmazeutische Zubereitung auf Basis von Lys-Plasminogen zur Verfügung zu stellen, die auch in hohen Dosen verabreicht werden kann, wobei die unerwünschten Nebenwirkungen minimiert oder vermieden werden. Solche Nebenwirkungen sind: cardiovaskuläre Erscheinungen, wie Gefäßdilatation, Blutdruckabfall, Steigerung des Atemwiderstandes (Bronchiokonstriktion), Beeinflussung der Herzfrequenz; weiters eine Aktivierung des Endothels, des Gerinnungssystems, der Thrombozyten, einschließlich des Kallikreinsystems mit Thrombingenerierung. Eine weitere Nebenwirkung ist die Freisetzung von aktivem Thrombin aus dem Thrombus während und nach der Lyse-Therapie. Eine weitere Nebenwirkung ist eine Stimulierung des Wachstums glatter Muskelzellen der Gefäßwand, was zu einer gesteigerten Reocclusionsneigung führt.

Die erfindungsgemäße Zubereitung soll in lyophilisierter Form vorliegen und zu einer gebrauchsfertigen Lösung rekonstituierbar sein.

Die erfindungsgemäße pharmazeutische Zubereitung auf Basis von Lys-Plasminogen, hergestellt aus Plasma oder auf Basis von rekombinant hergestelltem Lys-Plasminogen in lyophilisierter Form, ist gekennzeichnet durch einen Gehalt an einem Serin-Protease-Inhibitor und gegebenenfalls an einem Inhibitor-Kofaktor in einer Menge von 0,5 bis 30 nMol, vorzugsweise von 5 bis 25 nMol, pro mg Lys-Plasminogen. Die Erfindung beruht auf der Erkenntnis, daß die Applikation des Lys-Plasminogens zu keinen der angeführten Nebenwirkungen führt, wenn es zusammen mit einem Inhibitor und gegebenenfalls einem Inhibitor-Kofaktor in der oben angegebenen Menge verabreicht wird.

Bevorzugte Ausführungsformen der erfindungsgemäßen Zubereitung enthalten als Serin-Protease-Inhibitoren Aprotinin, Antithrombin III, $\alpha_1$-Antitrypsin, $\alpha_2$-Macroglobulin oder $C_1$-Esterase Inhibitor.

Als weitere bevorzugte Ausführungsform hat sich die Kombination von Antithrombin III als Serin-Protease-Inhibitor und einem Inhibitor-Kofaktor, vorzugsweise Heparin, erwiesen.

Die gebrauchsfertige Lösung der erfindungsgemäßen Zubereitung enthält vorteilhaft Lys-Plasminogen in einer Konzentration von 1 bis 50 mg, vorzugsweise 2,5 mg bis 25 mg pro ml der gebrauchsfertigen Lösung.

Die Erfindung umfaßt auch die Verwendung von Lys-Plasminogen in Verbindung mit einem Serin-Protease-Inhibitor zur Herstellung von Präparationen zur Behandlung oder Prophylaxe von Plasminogenmangelzuständen und Thrombosen und für die Herstellung von thromboseresistenten künstlichen Organen.

EP 0 480 906 B1

Das in der erfindungsgemäßen Zubereitung vorliegende Plasminogen weist auch ein besseres Adsorptionsverhalten an Blutgefäßen und künstlichen Oberflächen auf, wie weiter unten noch gezeigt wird.

Mit den nachfolgenden Beispielen wird die Erfindung noch näher erläutert.

## 1. Herstellung einer Lys-Plasminogen-Lösung

### Präparation 1

1 kg Cohn III-Niederschlag wurde bei 0°C in 10 l eines Phophatpuffers suspendiert, dem vorher 10 KIE/ml Aprotinin (1 KIE entspricht 0,022 nMol) zugesetzt worden waren. Nach einer Fällung mit Äthanol (10 %, -2°C) wurde zentrifugiert und der Überstand über ein Tiefenfilter auf Cellulose-Basis (AMF Cuno Zeta Plus 50 S) filtriert. Anschließend wurde mit 5 l Phophatpuffer verdünnt und 500 g Lysin-Polyacrylamidgel (hergestellt nach E.E. Rickli u. P.A. Cuendet, Biochem. Biophys. Acta 250, 447-451 (1971)) eingerührt. Nach einer Rührzeit von einer Stunde bei 0°C wurde das mit Plasminogen beladene Gel durch Filtration über Büchnertrichter abgetrennt und so lange mit einem Phosphatpuffer gewaschen, bis im Filtrat kein Protein mehr nachweisbar war. Durch Rühren mit einer Lösung von 6-Amino-Capronsäure (0,1 mol/l) in Phosphatpuffer wurde Plasminogen eluiert und durch Zusatz von Ammonsulfat (261 g je kg Eluat) ausgefällt.

Der durch Zentrifugation gewonnene Niederschlag wurde in einem isotonen Phosphat/Kochsalzpuffer gelöst und während 36 Stunden bei einer Temperatur von 5°C dialysiert; die Präparation enthielt 15 mg Plasminogen pro ml; in der SDS-PAGE wurde ein Gehalt an "Lys-Plasminogen" von 98 % gefunden.

### Präparation 2

Die Extraktion von Cohn III-Niederschlag mit Phosphatpuffer wurde nach Zusatz von 2 KIE/ml Aprotinin durchgeführt; die Fällung mit Äthanol, Zentrifugation und Filtration erfolgte analog Präparation 1. Nach der Verdünnung mit Phosphatpuffer wurde die Lösung über eine mit Lysin-Polyacrylamidgel gepackte Säule gepumpt. Waschung und Elution wurden mit den oben beschriebenen Lösungen, aber in der Säule durchgeführt. Nach der Fällung mit Ammonsulfat, Zentrifugation und dem Lösen des Niederschlages analog Präparation 1 wurde während 40 Stunden bei einer Temperatur von 7°C dialysiert; die Präparation enthielt 15 mg Plasminogen pro ml; in der SDS-PAGE wurde ein Gehalt an "Lys-Plasminogen" von 93 % gefunden.

### 2. Einfluß von Aprotinin auf Nebenreaktionen der Spontanlyse (in vivo)

Eine Lys-Plasminogenpräparation mit einem Gehalt von 500 CU (33 mg) wurde wie oben beschrieben hergestellt und mit 9 mg NaCl und mit 5000 (110 nMol) Einheiten Aprotinin (1 KIE entspricht 0,022 nMol; F. Markwardt, H. Landmann und H.P. Klöcking in: "Fibrinolytika und Antifibrinolytika", VEB Gustav Fischer Verlag, Jena (1972), S. 95-101) versetzt (diese Mengen beziehen sich auf 1 ml gebrauchsfertiger Lösung). Dies entspricht 3,3 nMol Inhibitor pro mg Lys-Plasminogen. Die Mischung verblieb eine Stunde bei Raumtemperatur. 2 ml obiger Lys-Plasminogenpräparation wurden pro kg einem narkotisierten Kaninchen als Bolus intravenös appliziert. Damit ergab sich eine Menge von 1000 Einheiten Lys-Plasminogen (66 mg) und 10 000 Einheiten Aprotinin pro kg Körpergewicht. Vor Applikation der Präparation, nach 30 min, 1 Std., 2 Std., 3 Std., 4 Std., 5 Std. nach Lys-Plasminogengabe wurden Blutproben gezogen und die Thrombocytenzahl im Blut mittels Coulter Counter (Coulter Electronics GmbH., Krefeld) gemessen. Als Vergleich wurde in parallel laufenden Versuchen die gleiche Menge Lys-Plasminogen ohne Aprotininzusatz injiziert. Die Ergebnisse sind in der nachstehenden Tabelle 1 zusammengefaßt:

## Tabelle 1

### Thrombocytenzahl G/l

|  | Lys-Plasminogen ohne Aprotinin | Lys-Plasminogen mit Aprotinin |
|---|---|---|
| vor Applikation | 366 | 395 |
| 30 min nach Appl. | 238 | 422 |
| 1 Std. nach Appl. | 174 | 369 |
| 2 Std. nach Appl. | 285 | 367 |
| 3 Std. nach Appl. | 245 | 342 |
| 4 Std. nach Appl. | 304 | 360 |
| 5 Std. nach Appl. | 325 | 376 |

Wie ersichtlich, kam es während des spontan einsetzenden fibrinolytischen Prozesses nach Applikation von Plasminogen, zu einer 52 % Verminderung von Thrombocyten. Diese unerwünschte Nebenreaktion des thrombolytischen Geschehens wurde durch die Kombination von Plasminogen und Aprotinin vermieden.

3. Einfluß von Antithrombin III/Heparin auf Nebenreaktionen der Spontanlyse (in vivo)

Eine Lys-Plasminogenpräparation mit 33 mg (500 CU) Plasminogen wurde mit 9 mg NaCl und mit einem Gemisch aus Antithrombin III und Heparin (1:6) versetzt, sodaß 25 U Antithrombin III (115 nMol) und 150 U Heparin zusätzlich pro ml Lösung resultierten. Dies entspricht 3,5 nMol Antithrombin III pro mg Lys-Plasminogen. Von dieser Mischung aus Wirkstoff und Inhibitoren wurden 2 ml/kg Körpergewicht einem narkotisierten Kaninchen verabreicht. Vor Applikation und nach Gabe von Lys-Plasminogen und den Inhibitoren wurde in bestimmten Zeitabständen Blut gezogen und die Thrombocyten im Coulter Counter gemessen. Ebenso wurde aus dem Plasma der Plasminogenspiegel amidolytisch bestimmt. Die Bestimmung erfolgt mittels chromogenem Plasminsubstrat S 2251 der Firma Kabi. In an sich bekannter Weise wurde mittels Überschuß von Aktivatoren Lys-Plasminogen zu Lys-Plasmin gespalten und in einem Trispuffermedium (pH = 7,4) die amidolytische Aktivität des Lys-Plasmins über die Freisetzung des Farbstoffes p-Nitroanilin gemessen. Aus diesen Werten wurde mittels Regressionsberechnung die Halbwertszeit des infundierten Lys-Plasminogens bestimmt. Verglichen wurde in einem parallel laufenden Versuch ohne Inhibitorzusatz. Die Ergebnisse sind in Tabelle 2 zusammengefaßt:

## Tabelle 2

| Lys-Plasminogen ohne Inhibitor | | Lys-Plasminogen mit AT III/Heparin | |
|---|---|---|---|
| Thrombocyten G/l | Plasminogen CU/ml | Thrombocyten G/l | Plasminogen CU/ml |
| vor Appl. 366 | 0,02 | 233 | 0,06 |
| 30 min nach Appl. 238 | 18 | 220 | 6,4 |
| 1 Std. nach Appl. 174 | 11,5 | 242 | 6,4 |
| 2 Std. nach Appl. 285 | 11 | 209 | 4,8 |
| 3 Std. nach Appl. 245 | 7 | 238 | 5,2 |
| 4 Std. nach Appl. 304 | - | 211 | 3,8 |
| 5 Std. nach Appl. 325 | 5 | 202 | 3,6 |
| Halbwertszeit | 129 min | | 296 min |

Wie ersichtlich, zeigte sich in den Kaninchen, die mit Lys-Plasminogen und Antithrombin III/Heparin behandelt wurden, daß einerseits der spontane Abfall von Thrombocyten als Nebenreaktion der Thrombolyse vermieden wurde, andererseits die Halbwertszeit für Lys-Plasminogen im Plasminogen-Inhibitorgemisch verlängert wurde.

4. Einfluß von CI-Inhibitor auf Nebenreaktionen der der Spontanlyse (in vivo)

Eine Lys-Plasminogenpräparation enthaltend per ml 500 CU (= 33 mg) Lys-Plasminogen und 9 mg NaCl wurde mit 125 U (= 212 nMol) CI-Inhibitor versetzt. Das entspricht 6,4 nMol CI-Inhibitor per mg Lys-Plasminogen (1 E entspricht 1,7 nMol; P.C. Harpel in: "Proc. int. Workshop on Protein Separation and Plasma Fractionation"; Ed. H.E. Sandberg (1977); S. 289-302). An einem narkotisierten Kaninchen wurden 2 ml obiger Lösung intravenös injiziert. Vor und nach der Applikation wurde Blut abgenommen. Als Vergleich diente eine Lys-Plasminogenpräparation ohne Inhibitorzusatz. Es wurde die Gerinnungsfähigkeit des Vollblutes mittels eines Thromboelastographen (Fa. Hellige) gemessen. Gemessen wurde in vitro die Reaktionszeit bis zur Gerinnselbildung nach Zusatz von $CaCl_2$ zu einem citratstabilisierten Vollblut. Die Ergebnisse sind in der Tabelle 3 zusammengefaßt:

## Tabelle 3

### Gerinnungszeit in min

| | Lys-Plasminogen ohne Inhibitor | Lys-Plasminogen + Cl-Inhibitor |
|---|---|---|
| vor Applikation | 18 | 15 |
| 1 Std. nach Appl. | 21 | 16 |
| 2 Std. nach Appl. | | 12 |
| 3 Std. nach Appl. | keine Gerinnung | 13 |
| 4 Std. nach Appl. | > 60 min | 12 |
| 5 Std. nach Appl. | | 10 |

Die Aktivierung des fibrinolytischen Systems stellt eine schwere Belastung des Körpers dar. Im Extremfall kann es zu schweren Blutungskomplikationen durch Ungerinnbarkeit des Blutes kommen. Das zeigte sich im Falle der Applikation von Lys-Plasminogen ohne Inhibitoren. Wird jedoch Lys-Plasminogen mit Cl-Inhibitor injiziert, so können Zwischenfälle wie Blutungskomplikationen vermieden werden.

### 5a. Einfluß auf den Atemdruck beim Meerschweinchen

Appliziert man in einem narkotisierten Meerschweinchen 66 mg (1000 U) Lys-Plasminogen/kg intravenös und beginnt nach einer Stunde mit der Thrombolysetherapie durch Applikation von 200 000 Einheiten Urokinase (UK) pro kg Körpergewicht, so zeigt sich ein spontaner Anstieg des Atemdrucks nach UK-Gabe. Wird jedoch Lys-Plasminogen mit $\alpha_2$-Macroglobulin vorgemischt und in einem Verhältnis von 230 mg $\alpha_2$-Macroglobulin (= 317 nMol) und 1000 Einheiten (66 mg) Lys-Plasminogen pro kg Körpergewicht (entspricht 4,8 nMol $\alpha_2$-Macroglobulin per mg Lys-Plasminogen) appliziert und nach 60 min Latenzzeit ebenfalls mit 200 000 Einheiten UK/kg die Therapie begonnen, so findet kein Atemdruckanstieg nach Applikation von UK statt.

### Tabelle 4
### Atemdruck (in % des Ausgangswertes)

| | vor Appl. von UK | nach Appl. von UK | Variations-koeffizient |
|---|---|---|---|
| Lys-Plasminogen | 100 % | 140 % (rel.) | 10 % |
| Lys-Plasminogen + $\alpha_2$M | 100 % | 93 % (rel.) | 20 % |

### 5b. Einfluß auf den Blutdruck beim Meerschweinchen

In ähnlicher Weise zeigten sich unerwünschte Nebenreaktionen auch im spontanen Abfall des Blutdrucks. Wie oben beschrieben, wurde am Meerschweinchen-Modell der Blutdruck während der Thrombolysetherapie mit Urokinase und Lys-Plasminogen verfolgt.

Es kam bei Applikation von 1000 Einheiten (66 mg) Lys-Plasminogen/kg zu keiner signifikanten Änderung des Blutdruckes. Nach Applikation von 200 000 UK/kg kam es zu einem spontanen Abfall der bis Ende des Versuches (etwa 20 min) anhielt. Eine Erholungsphase konnte nicht beobachtet werden. Wurde jedoch Lys-Plasminogen mit CI-Inhibitor vorgemischt und diese Mischung von 1000 Einheiten Lys-Plasminogen (66 mg) und 1000 Einheiten CI-Inhibitor (1700 nMol) pro kg appliziert (entspricht 25 nMol CI-Inhibitor pro mg Lys-Plasminogen) mit anschließender UK-Gabe, so zeigte sich zwar ein geringer Blutdruckabfall, der aber nach etwa 5 min den Ausgangswert erreichte und sich normalisierte.

5c. Einfluß auf Atemdruck beim Meerschweinchen

Im gleichen Tiermodell wurde die Kombination Lys-Plasminogen [$\alpha_2$-Macroglobulin] CI-Inhibitor getestet. 230 mg $\alpha_2$-Macroglobulin wurden mit 1000 Einheiten CI-Inhibitor und 1000 Einheiten (66 mg) Lys-Plasminogen vorgemischt und dieses Gemisch pro kg Meerschweinchen injiziert. Es kam nach UK-Gabe von 200 000 Einheiten/kg zu keinem Anstieg des Atemdrucks.

6. Adsorption von Lys-Plasminogen

a) Adsorption an Blutgefäßen

Eine Lys-Plasminogen-Lösung mit Inhibitorgehalt (Aprotinin, CI-Esterase-Inhibitor ) von 0,6 nMol/mg Plasminogen wurde in ihrem Adsorptionsverhalten an einer Kaninchenaorta getestet. Als Vergleich dazu diente das native Glu-Plasminogen ohne Inhibitorzusatz.

Eine Aorta wurde einem getöteten Kaninchen entnommen und umgestülpt in Stücken auf Glasstäben aufgezogen. (Lys- und Glu-) Plasminogenlösungen wurden in einer Tyrodelösung vorbereitet und die Aorten mit etwa 1 cm$^2$ Oberfläche mit 0,5 ml Plasminogen-Lösung mit einer Konzentration von 0,015 mg Plasminogen pro ml bei 37°C 10 min, 30 min und 60 min inkubiert. Nach der Kontaktzeit wurden die Aortenstücke aus den Plasminogen-Lösungen entfernt und mit Tyrodelösung gewaschen. Die mit Plasminogen adsorbierten Aorten wurden in eine Gefäß gebracht, das 500 myl Tyrodelösung, 50 myl Urokinase (Fa. Medac) mit 25.000 IE/ml, 50 myl chromogenes Substrat S 2251 (Fa. Kabi) enthielt. Bei 37°C wurde 10 min inkubiert. Dabei wurde an der Aorta adsorbiertes Plasminogen mittels UK in Lys-Plasmin umgewandelt. Die Spaltungsraten, mit denen Lys-Plasmin vom chromogenen Substrat P-Nitroanilin abspaltet, wurde photometrisch vermessen, nachdem mit 400 myl Eisessig die Reaktionen gestoppt worden waren.

# Tabelle 5

$$nMol/min.cm^2$$

|  | Lys-Plasminogen (incl. Inhibitor) | Glu-Plasminogen (nativ) |
|---|---|---|
| Kontaktzeit Plasminogen - Aorta |  |  |
| 10 min | 0,56 | 0,03 |
| 30 min | 1,2 | 0,2 |
| 60 min | 2,1 | 0,16 |

Die Lys-Plasminogen-Präparation adsorbiert viel stärker an das Blutgefäß und läßt sich mit Aktivator in Plasmin überführen, die Inhibitoren im Präparat verhindern weder die Adsorption noch den Aktivierungsschritt.

b) Adsorption an künstlichen Oberflächen

Lys-Plasminogen mit einem Inhibitorgehalt von 0,6 nMol/mg Plasminogen wird in seinem Adsorptionsverhalten an künstlichen Oberflächen getestet. Als Vergleich dazu dient ein natürlich vorkommendes Glu-Plasminogen ohne Zusatz von Inhibitoren.

Sowohl Lys-Plasminogen als auch Glu-Plasminogen werden in physiologischen NaCl-Lösungen auf eine Konzentration von 4 mg Plasminogen pro ml gestellt. 250 myl der Probe werden in Näpfchen pipettiert. In die vollgefüllten Näpfchen werden Plastikplättchen (Kämme) gehängt (Polystyrol 158K der Fa. BASF), an deren Oberfläche eine Adsorption von Plasminogen stattfindet. Nach 60 min, 120 min Kontaktzeit bei Raumtemperatur werden die Plastikplättchen entfernt, gewaschen und in ein Reaktionsgemisch mit 100 myl Trispuffer, pH 7,2, 50 myl Urokinase (Fa. Medac) 25.000 IE/ml, und 50 myl chromogenes Substrat S 2251 (Fa. Kabi) gebracht. Nach 10 min Raumtemperatur wird 50 myl Eisessig zugesetzt und die Reaktionen gestoppt. Adsorbiertes Plasminogen wird mit UK in Plasmin umgewandelt, das das chromogene Substrat spaltet. Die Umsetzung des chromogenen Substrates läßt auf die Adsorption an künstlichen Oberflächen schließen.

## Tabelle 6

$$nMol/min. \ cm^2$$

|  | Lys-Plasminogen (incl. Inhibitor) | Glu-Plasminogen (nativ) |
|---|---|---|
| Kontaktzeit |  |  |
| 60 min | 0,23 | 0,09 |
| 120 min | 1,2 | 0,19 |

Auch hier wird gezeigt, daß Lys-Plasminogen mit Inhibitoren deutlich besser und schneller adsorbiert als Glu-Plasminogen ohne Inhibitoren und auch aktivierbar ist, ohne Einfluß der im Präparat enthaltenen Inhibitoren.

7. Herstellung der erfindungsgemäßen pharmazeutischen Zubereitung

Nachfolgend ist die Herstellung zweier erfindungsgemäßer pharmazeutischer Zubereitungen, ausgehend von den in Präparation 1 und Präparation 2 erhaltenen Dialysaten, beschrieben. Die beiden Zubereitungen unterscheiden sich in ihrem Gehalt an Inhibitoren.

a) 32 ml des Dialysates der Lys-Plasminogenpräparation 1 mit einem Gehalt von 15 mg Lys-Plasminogen pro ml wurden mit 6,4 ml einer $C_1$-Esterase-Inhibitorpräparation ($C_1$ Esterase Inhibitor STIM 3 Human Haemoderivate 500 Einheiten pro 10 ml) versetzt. Anschließend wurden 20 mg Aprotinin zugesetzt, wodurch sich eine Konzentration von 10 Einheiten ( = 17 nMol) $C_1$-Esterase-Inhibitor und 4469 KIE ( = 98 nMol) Aprotinin pro ml Lys-Plasminogen-Lösung mit 15 mg Lys-Plasminogen ergab. Daraus berechnet sich ein Inhibitorgehalt von 7,7 nMol pro mg Lys-Plasminogen. Die Lösung verblieb mindestens 15 min bei 4°C, wurde mit 20 mmol Lysin pro Liter versetzt, auf einen pH-Wert von 7 gestellt, lyophilisiert, zwecks Virusinaktivierung hitzebehandelt, gelöst, sterilfiltriert, abgefüllt und im Endbehältnis lyophilisiert.

b) 32 ml des Dialysates der Lys-Plasminogenpräparation 2 mit einem Gehalt von 15 mg Lys-Plasminogen pro ml wurden mit 1280 ml einer $C_1$-Esterase-Inhibitorpräparation (siehe oben) und 1,79 mg Aprotinin versetzt und wie oben beschrieben aufgearbeitet. Bei dieser Zubereitung ergab sich ein Inhibitorgehalt von 0,81 nMol pro mg Lys-Plasminogen.

8. Anwendungen

Personen mit angeborenen Plasminogenmangelzuständen, die sowohl durch quantitative als auch qualitative Störungen gekennzeichnet sind, haben ein erhöhtes Risiko, an thrombotischen Zuständen zu erkranken. Oft werden diese thrombotischen Ereignisse durch äußere Umstände, wie Traumen, Operationen, Schwangerschaften, Infektionen, entzündliche Geschehen, ausgelöst.

Für Behandlung und Prophylaxe dieser Plasminogenmangel-Patienten kann Lys-Plasminogen 2 bis 4 mal täglich in Dosen von 10 bis 100 E/kg infundiert werden. Lys-Plasminogen kann auch für Prophylaxe und Therapie, entweder allein oder in Kombination eines Aktivators, für eine Reihe von erworbenen Erkrankungen, wie disseminierte intravaskuläre Gerinnung, periphere arterielle Thrombose, Myokardthrombose, Insult, Myokardinfarkt, eingesetzt werden. Die systemische oder lokale (intracoronare) Thrombolyse mittels Urokinase, Streptokinase, Tissue-Plasminogen-Aktivator ist heute etablierte Therapie eines Myokardinfarktes.

Der Erfolg der Lyserate ist allerdings abhängig vom Alter des Thrombus, und in vielen Fällen bleiben die Coronarthromben, wegen zu später Hospitalisierung, lyseresistent. Die Applikation der erfindungsgemäßen Zubereitung, entweder systemisch oder lokal (auch in Körperhöhlen) kann eine Anreicherung von Lys-Plasminogen am Thrombus bewirken und kann daher die Lyserate der anschließenden Aktivator-Proaktivator-Applikation erhöhen.

Die erfindungsgemäße Zubereitung kann sofort bei Einsetzen der klinischen Symptomatik oder bei Verdacht auf Myokardinfarkt, noch vor der Diagnosesicherung, entweder intravenös, subkutan oder peroral appliziert werden und sich in dem sich noch in Bildung befindlichen thrombus anreichern. Damit wird eine Verlängerung des Zeitintervalles erreicht, in welchem fibrinolytisch wirksame Aktivatoren eine effektvolle Thrombolyse erreichen.

Ein häufiges Problem der Thrombolyse besteht in der Reokklusion des Gefäßes nach erfolgter Öffnung. Die lokale oder systemische Applikation nach erfolgter Thrombolyse kann ein filmartiges Überziehen der geschädigten oder arterosklerotisch veränderten Gefäßoberfläche mit Lys-Plasminogen bewirken und durch die so hergestellte thromboseresistente Oberfläche die Reokklusion verhindern.

Die nach angioplastischen Eingriffen beobachtete Restenose wird durch Applikation der erfindungsgemäßen Zubereitung vor, während und nach der Angioplastik verhindert.

Die gegenüber konventionellen Therapien resistente tiefe Beinvenenthrombose kann durch Applikation distal von der Thrombose, bei gleichzeitiger systemischer Lysetherapie, erfolgreich behandelt werden.

Die Herstellung thromboseresistenter Gefäßprothesen wird durch Beschichtung der Oberfläche mit Lys-Plasminogen erreicht; ebenso eignet sich Lys-Plasminogen für die Herstellung künstlicher thromboseresistenter Organe, z.B. Herzklappe, künstliche Gelenke. Lys-Plasminogen kann mit Proenzymen und/oder Enzymen gemischt werden. diese in vitro hergestellten Präparationen entfalten ihre thrombolytischen Wirkungen nach Applikation in vivo.

Das mit der erfindungsgemäßen Zubereitung verabreichte Lys-Plasminogen mit anschließender Aktivatorbehandlung (z.B. Urokinase) verringert das Reokklusionsrisiko.

Patienten mit Fettstoffwechselstörungen, insbesondere mit erhöhtem Lp (a) sind durch schwere Artherosklerose, beding durch fehlerhaftes Resorptionsverhalten ihrer Gefäßwand gekennzeichnet. Die Substitution durch Lys-Plasminogen verhindert bei diesen Patienten aufgrund der strukturellen Ähnlichkeit die Aufnahme von Lp (a) in die Gefäßwand, verlangsamt den artherosklerotischen Prozeß und macht ihn reversibel.

**Patentansprüche**
**Patentansprüche für folgende Vertragsstaaten : AT, BE, CH, DE, DK, FR, GB, IT, LI, NL, SE**

1.  Pharmazeutische Zubereitung auf Basis von Lys-Plasminogen aus Plasma oder rekombinant hergestelltem Lys-Plasminogen in lyophilisierter Form mit einem Gehalt eines Serin-Protease-Inhibitors und gegebenenfalls eines Inhibitor-Kofaktors in einer Menge von 0,5 bis 30 nMol, vorzugsweise von 5 bis 25 nMol, pro mg Lys-Plasminogen.

2.  Pharmazeutische Präparation nach Anspruch 1, dadurch gekennzeichnet, daß der Serin-Protease-Inhibitor ausgewählt ist aus der Gruppe umfassend Aprotinin, $\alpha_2$-Macroglobulin, $\alpha_1$-Antitrypsin, Antithrombin III und Cl-Esterase-Inhibitor, humanen, tierischen oder rekombinanten Ursprungs.

3.  Pharmazeutische Präparation nach Anspruch 1, dadurch gekennzeichnet, daß als Serin-Protease-Inhibitor Antithrombin III und als Inhibitor-Kofaktor vorzugsweise Heparin vorgesehen sind.

4. Pharmazeutische Zubereitung nach einem oder mehreren der Ansprüche 1 bis 3, dadurch gekennzeichnet, daß sie zu einer gebrauchsfertigen Lösung rekonstituierbar ist, welche Plasminogen in einer Konzentration von 1 mg bis 50 mg, vorzugsweise 2,5 mg bis 25 mg pro ml der gebrauchsfertigen Lösung enthält.

5. Verwendung von Lys-Plasminogen in Verbindung mit einem Serin-Protease-Inhibitor zur Herstellung von Präparationen zur Behandlung oder Prophylaxe von Plasminogenmangelzuständen und Thrombosen und für die Herstellung von thromboseresistenten künstlichen Organen.

**Patentansprüche für folgenden Vertragsstaat : ES**

1. Verfahren zur Herstellung einer pharmazeutischen Zusammensetzung auf Basis von Lys-Plasminogen zur Behandlung und Prophylaxe von Plasminogen-Mangelzuständen und Thrombosen und für die Herstellung von thromboseresistenten künstlichen Organen, wobei die Zusammensetzung aus Lys-Plasminogen und einem Serinprotease-Inhibitor in einer Menge von 0,5 bis 30 nMol Serinprotease-Inhibitor pro mg Lys-Plasminogen und gegebenenfalls einen Inhibitor-Kofaktor enthält, welches Verfahren dadurch gekennzeichnet ist, daß es die folgenden Verfahrensschritte aufweist:
(a) Mischen einer Lösung von Lys-Plasminogen mit dem Inhibitor-Kofaktor für den Fall, daß diese Komponente der Zusammensetzung zugegeben werden soll, und anschließendem Zugeben eines Serinprotease-Inhibitors in der angegebenen Menge.
(b) Filtration durch ein Sterilfilter,
(c) Lyophilisieren des Produkts aus dem obigen Schritt,
(d) gegebenenfalls Vornehmen eines Virus-Inaktivierungsschrittes und Füllen des Produkts in die Endbehälter, mit denen es vermarktet wird.

2. Verfahren nach Anspruch 1, wobei Lys-Plasminogen aus Plasma gewonnen wird.

3. Verfahren nach Anspruch 1, wobei das Lys-Plasminogen rekombinantes Lys-Plasminogen ist.

4. Verfahren nach Anspruch 1, wobei die Zusammensetzung einen Serinprotease-Inhibitor in einer Menge von 5 bis 24 nMol pro mg Lys-Plasminogen enthält.

5. Verfahren nach Anspruch 1, wobei die Zusammensetzung einen Inhibitor-Kofaktor enthält.

6. Verfahren nach Anspruch 1, wobei der Serinprotease-Inhibitor ausgewählt ist aus der Gruppe bestehend aus Aprotinin, $\alpha_2$-Macroglobulin, $\alpha_1$-Antitrypsin, Antithrombin III und $C_1$-Esterase-Inhibitor von menschlicher, tierischer oder rekombinanter Herkunft.

7. Verfahren nach Anspruch 4, wobei der Serinprotease-Inhibitor Antithrombin III umfaßt.

8. Verfahren nach Anspruch 7, wobei die Zusammensetzung weiters Heparin als Inhibitor-Kofaktor enthält.

9. Verfahren nach Anspruch 1, wobei die erhaltene Zusammensetzung zu einer gebrauchsfertigen Lösung rekonstituierbar ist, welche Lys-Plasminogen in einer Konzentration von 1 mg bis 50 mg pro ml der gebrauchsfertigen Lösung enthält.

10. Verfahren nach Anspruch 9, wobei die Zusammensetzung Lys-Plasminogen in einer Konzentration von 2,5 mg bis 25 mg pro ml der gebrauchsfertigen Lösung enthält.

**Claims**
**Claims for the following Contracting States : AT, BE, CH, DE, DK, FR, GB, IT, LI, NL, SE**

1. A pharmaceutical preparation based on Lys-plasminogen from plasma or on recombinantly produced Lys-plasminogen in lyophilised form and having a content of a serine-protease inhibitor and optionally of an inhibitor co-factor in an amount of from 0.5 to 30 nmol, preferably from 5 to 25 nmol, per mg of Lys-plasminogen.

2. A pharmaceutical preparation according to claim 1, characterised in that the serine-protease inhibitor is selected from the group comprising aprotinin, $\alpha_2$-macroglobulin, $\alpha_1$-antitrypsin, antithrombin III and C1-esterase inhibitor of human, animal or recombinant origin.

3. A pharmaceutical preparation according to claim 1, characterised in that antithrombin III is provided as the serine-protease inhibitor and heparine is preferably provided as the inhibitor co-factor.

4. A pharmaceutical preparation according to one or several of claims 1 to 3, characterised in that it can be reconstituted to a ready-for-use solution, which contains plasminogen at a concentration of from 1 mg to 50 mg, preferably 2.5 mg to 25 mg, per ml of the ready-for-use solution.

5. Use of Lys-plasminogen in combination with a serine-protease inhibitor for producing preparations for the treatment or prophylaxis of plasminogen deficiency conditions and thromboses and for the production of thromboresistant artificial organs.

**Claims for the following Contracting State : ES**

1. A method of producing a pharmaceutical composition based on Lys-plasminogen for the treatment and prophylaxis of plasminogen deficiency conditions and thromboses and for the production of thromboresistant artificial organs, wherein the composition comprises Lys-plasminogen and a serine-protease inhibitor in an amount of from 0.5 to 30 nmol of serine-protease inhibitor per mg Lys-plasminogen and optionally an inhibitor co-factor, which method is characterised in that it comprises the following method steps:
   (a) mixing a solution of Lys-plasminogen with the inhibitor co-factor in case that this component is to be added to the composition, and subsequently adding a serine-protease inhibitor in the amount stated,
   (b) filtering through a sterile filter,
   (c) lyophilising the product obtained from the above step,
   (d) optionally carrying out a virus inactivating step and filling the product into the final containers in which it is marketed.

2. A method according to claim 1, wherein Lys-plasminogen is recovered from plasma.

3. A method according to claim 1, wherein the Lys-plasminogen is recombinant Lys-plasminogen.

4. A method according to claim 1, wherein the composition contains a serine-protease inhibitor in an amount of from 5 to 24 nmol per mg Lys-plasminogen.

5. A method according to claim 1, wherein the composition contains an inhibitor co-factor.

6. A method according to claim 1, wherein the serine-protease inhibitor is selected from the group consisting of aprotinin, $\alpha_2$-macroglobulin, $\alpha_1$-antitrypsin, antithrombin III and $C_1$-esterase inhibitor of human, animal or recombinant origin.

7. A method according to claim 4, wherein the serine-protease inhibitor comprises antithrombin III.

8. A method according to claim 7, wherein the composition further contains heparin as the inhibitor co-factor.

9. A method according to claim 1, wherein the composition obtained can be reconstituted to a ready-for-use solution containing Lys-plasminogen at a concentration of from 1 mg to 50 mg per ml of the ready-for-use solution.

10. A method according to claim 9, wherein the composition contains Lys-plasminogen at a concentration of from 2.5 mg to 25 mg per ml of the ready-for-use solution.

**Revendications**

**Revendications pour les Etats contractants suivants : AT, BE, CH, DE, DK, FR, GB, IT, LI, NL, SE**

1. Préparation pharmaceutique à base de lys-plasminogène de plasma ou de lys-plasminogène produit par génie génétique, sous forme lyophilisée, contenant un inhibiteur de la sérine-protéase et le cas échéant un cofacteur inhibiteur en une quantité de 0,5 à 30 nmol, de préférence de 5 à 25 nmol, par mg de lys-plasminogène.

2. Préparation pharmaceutique selon la revendication 1, caractérisée en ce que l'inhibiteur de la sérine-protéase est sélectionné dans le groupe comprenant l'aprotinine, l'$\alpha_2$-macroglobuline, l'$\alpha_1$-antitrypsine, l'antithrombine III et l'inhibiteur de la $C_1$-estérase, d'origine humaine, animale ou génétique.

3. Préparation pharmaceutique selon la revendication 1, caractérisée en ce que sont prévues l'antithrombine III comme inhibiteur de la sérine-protéase et de préférence l'héparine comme cofacteur inhibiteur.

4. Préparation pharmaceutique selon une ou plusieurs des revendications 1 à 3, caractérisée en ce qu'elle est reconstituable en une solution prête à l'emploi contenant du plasminogène en une concentration de 1 mg à 50 mg, de préférence 2,5 mg à 25 mg par ml de solution prête à l'emploi.

5. Utilisation de lys-plasminogène en association avec un inhibiteur de la sérine-protéase pour produire des préparations destinées au traitement ou à la prévention des états de déficit en plasminogène et des thromboses et pour la fabrication d'organes artificiels thromborésistants.

**Revendications pour l'Etat contractant suivant : ES**

1. Procédé de préparation d'une composition pharmaceutique à base de lys-plasminogène destinée au traitement ou la prévention des états de déficit en plasminogène et des thromboses et à la fabrication d'organes artificiels thromborésistants, la composition étant constituée de lys-plasminogène et d'un inhibiteur de la sérine-protéase en une quantité de 0,5 à 30 nmol d'inhibiteur de sérine-protéase par mg de lys-plasminogène et contenant le cas échéant un cofacteur inhibiteur, lequel procédé est caractérisé en ce qu'il comporte les étapes suivantes :
   a) mélange d'une solution de lys-plasminogène avec le cofacteur inhibiteur pour le cas où ce composant devrait être ajouté à la composition et addition subséquente d'un inhibiteur de la sérine-protéase dans la quantité indiquée,
   b) filtration sur un filtre stérie,
   c) lyophilisation du produit provenant de l'étape ci-dessus,
   d) le cas échéant mise en oeuvre d'une étape d'inactivation virale et introduction du produit dans les récipients finals dans lesquels il est commercialisé.

2. Procédé selon la revendication 1, le lys-plasminogène étant obtenu à partir de plasma.

3. Procédé selon la revendication 1, le lys-plasminogène étant du lys-plasminogène recombiné.

4. Procédé selon la revendication 1, la composition contenant un inhibiteur de la sérine-protéase en une quantité de 5 à 24 nmol par mg de lys-plasminogène.

5. Procédé selon la revendication 1, la composition contenant un cofacteur inhibiteur.

6. Procédé selon la revendication 1, l'inhibiteur de la sérine-protéase étant sélectionné dans le groupe comprenant l'aprotinine, l'$\alpha_2$-macroglobuline, l'$\alpha_1$-antitrypsine, l'antithrombine III et l'inhibiteur de la $C_1$-estérase, d'origine humaine, animale ou génétique.

7. Procédé selon la revendication 4, dans lequel l'inhibiteur de la sérine-protéase comprend l'antithrombine III.

8. Procédé selon la revendication 7, la composition contenant en outre de l'héparine comme cofacteur inhibiteur.

9. Procédé selon la revendication 1, la composition obtenue étant reconstituable en une solution prête à l'emploi contenant du lys-plasminogène en une concentration de 1 mg à 50 mg par ml de solution prête à l'emploi.

10. Procédé selon la revendication 9, la composition contenant du lys-plasminogène en une concentration de 2,5 mg à 25 mg par ml de solution prête à l'emploi.